(19) Europäisches Patentamt / European Patent Office / Office européen des brevets

(11) **EP 3 436 767 B1**

(12) **EUROPÄISCHE PATENTSCHRIFT**

(45) Veröffentlichungstag und Bekanntmachung des Hinweises auf die Patenterteilung:
**13.05.2020 Patentblatt 2020/20**

(21) Anmeldenummer: **17718732.5**

(22) Anmeldetag: **31.03.2017**

(51) Int Cl.:
*G01J 3/50* (2006.01)  *G01N 21/952* (2006.01)
*G01B 7/12* (2006.01)  *G01B 7/02* (2006.01)
*G01B 11/10* (2006.01)  *G01B 11/08* (2006.01)

(86) Internationale Anmeldenummer:
**PCT/IB2017/000372**

(87) Internationale Veröffentlichungsnummer:
**WO 2017/168237 (05.10.2017 Gazette 2017/40)**

(54) **KOMBINATIONSSSENSOR**

COMBINATION SENSOR

CAPTEUR MIXTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.04.2016 EP 16163593**

(43) Veröffentlichungstag der Anmeldung:
**06.02.2019 Patentblatt 2019/06**

(73) Patentinhaber: **Schleuniger Holding AG**
**3608 Thun (CH)**

(72) Erfinder:
• **Deschler, Raphael**
**CH-3653 Oberhofen (CH)**
• **JOST, Michael**
**3600 Thun (CH)**
• **HUGGLER, Chistian**
**3612 Steffisburg (CH)**
• **HOFER, Reto**
**3705 Faulensee (CH)**

(74) Vertreter: **Patentbüro Paul Rosenich AG**
**BGZ**
**9497 Triesenberg (LI)**

(56) Entgegenhaltungen:
EP-A2- 0 692 697  CN-A- 1 403 821
CN-U- 87 214 470  DE-A1- 10 219 848
DE-A1-102006 010 992  GB-A- 2 160 654
JP-A- 2000 161 985

**EP 3 436 767 B1**

**Beschreibung**

Die Erfindung betrifft eine Anordnung zur automatischen, berührungslosen Erkennung von länglichen Objekten wie Kabeln, Drähten oder Profilen, mit zumindest einem induktiven Messsystem und zumindest einem optischen Messsystem für das Objekt in einem gemeinsamen Gehäuse, gemäss dem Oberbegriff von Anspruch 1, sowie eine Anlage gemäss dem Oberbegriff von Anspruch 16 unter Verwendung einer solchen Anordnung.

In Bearbeitungsmaschinen für Kabel, Drähte oder ähnliche längliche Objekte muss durch sichere Erkennung der Objekte sichergestellt werden, dass die Art der Bearbeitung, die Einstellungen der Maschine und deren Parameter auf das jeweilige Objekt und die vom Benutzer definierten Anforderungen abgestimmt sind. Dazu sind vorzugsweise vor oder am Eingang der Bearbeitungsmaschine Messsysteme angeordnet, die eine zuverlässige Identifikation des länglichen Objekts ermöglichen sollen. Diese umfassen mehrere Sensoranordnungen, da die Zuverlässigkeit der Identifikation mit der Anzahl unterschiedlicher Messprinzipien steigt (weil beispielsweise Kabel mit gleichem Aussendurchmesser und gleicher Farbe verschiedene interne leitende Konfigurationen aufweisen können oder Kabel mit unterschiedlichem Aufbau - Feinlitze, Litze, Draht - bei unterschiedlichem Kupferquerschnitt die gleiche elektromagnetische Charakteristik aufweisen können).

In der DE10219848A1 ist ein berührungsloses Zentrizitäts- und Durchmesser-Messsystem offenbart, mit einer optischen Messvorrichtung zur Bestimmung des Aussendurchmessers und der Lage eines länglichen Objektes in einer optischen, senkrecht und quer zur Mittelachse einer Messeinrichtung angeordneten Messebene. Das Objekt weist einen Leiter und eine diesen isolierende Umhüllung auf, wobei die Lage des Leiters in einer induktiven Messebene mittels einer induktiven Messspulenvorrichtung ermittelt wird, welche Messebene ebenfalls senkrecht und quer zur Mittelachse der Messeinrichtung angeordnet ist. Die durch die optische Messvorrichtung bestimmte Lage des Objekts wird mit der durch die induktive Messspulenvorrichtung ermittelten Lage des Leiters in Beziehung gesetzt und daraus die Zentrizität des Leiters in der Umhüllung errechnet. Dabei sind die Messspulen der Messspulenvorrichtung paarweise oder hälftig bezüglich der optischen Messebene angeordnet, werden aber vom länglichen Objekt nicht durchquert. Das Messobjekt selbst verbleibt immer ausserhalb der Messspulen. Die paarweise Anordnung der Spulen dient der differenziellen Messung der Feldstärke, ausgehend vom im Leiter fliessenden Wechselstrom, der mittels eines zusätzlichen Induktors des Messsystems im Leiter induziert wird. Somit ist die Differenz der in den Spulen induzierten Spannungen ein Mass der Exzentrizität des Leiters bezüglich der Spulen-Spiegelachse. Die Spulen sind also nicht Teil eines Schwingkreises der vom Kupfer gedämpft wird. Überdies ist kein weiteres optisches Messsystem vorhanden.

Die WO2009150620A1 beschreibt wiederum eine Anordnung von Sensoren, die aufgrund von Messungen des Aussendurchmessers eines Kabels, des Durchmessers des innerhalb des isolierenden Mantels liegenden metallischen Leiters und gegebenenfalls weiterer von aussen detektierbarer Merkmale eine automatische oder halbautomatische Identifikation des jeweiligen zur Bearbeitung vorgesehenen Kabels oder zumindest Kabeltyps gestattet. Die verschiedenen genannten Sensoren sind funktional unabhängig voneinander, weisen keinerlei synergistische Effekte auf, weisen keinerlei gemeinsam genutzte Elemente oder Bereiche auf und bilden daher keinen Verbund von Messsystemen. CN 1 403 821 A offenbart eine Anordnung zur automatischen Erkennung von länglichen Objekten. EP 0 692 697 A2 offenbart eine Vorrichtung zur Positionsbestimmung eines Leiters innerhalb eines isolierenden Mantels eines elektrischen Kabels mit elektrisch induktiven sowie optischen Mitteln. Dabei wird die Exzentrizität des Leiterkerns in Bezug auf die gewünschte Position innerhalb des Kabels bestimmt. GB 2 160 654 A offenbart eine Anordnung zur Messung der Dicke einer Schicht. JP 2000 161985 A offenbart einen Positionssensor für die Ermittlung der axialen Position einer Welle. DE 10 2006 010 992 A1 offenbart eine Vorrichtung und ein Verfahren zur Erfassung der Position eines durch eine Auswertefläche fliegenden Gegenstands, insbesondere eines Projektils oder Pfeils, insbesondere für Schiessstände bzw. Sportschützen. Aus dieser Druckschrift ist bekannt, das Licht einer Lichtquelle - in Form eines Lichtvorhanges - durch die Auswertefläche, anschließend durch eine Linse und zu einem hinter der Linse angeordneten Spiegel zu senden. Der Spiegel reflektiert das Licht, sodass dieses wieder durch die Linse und durch die Auswertefläche geleitet und einer Kameraeinrichtung zugeführt wird.

Es war die Aufgabe der vorliegenden Erfindung, eine verbesserte SensorAnordnung zu finden, welche Synergien verschiedener Messsysteme nutzt, damit sich eine kompakte, funktional robuste Bauweise eines Kombinationssensors ergibt, der die Ermittlung mehrere Charakteristika des Objekts erlaubt, um daraus eine rasche und zuverlässige Erkennung des Objekts zu ermöglichen.

Zur Lösung dieser Aufgabe ist die eingangs beschriebene Anordnung dadurch gekennzeichnet, dass das induktive Messsystem als Wirbelstromsensor zur Bestimmung einer elektromagnetischen Charakteristik des Objektes ausgelegt ist und zwei in Serie geschaltete, gleichsinnig orientierte und koaxiale Halbspulen aufweist, die in axialer Richtung einen Abstand voneinander aufweisen, koaxial zur Längsachse das Objekt umwinden, so dass deren Innenraum ein induktives zylinderförmiges Messvolumen definiert, wobei die Halbspulen zusammen mit einer elektrisch parallel dazu geschalteten Kapazität einen als induktiven Sensor des induktiven Messsystems genutzten Parallelschwingkreis bilden, der mit einer elektronischen Auswerteschaltung verbunden ist, und dass das optische scheibenförmige Messvolumen (DCPv) durch den Abstand der Halbspulen (E1a, E1b) und die Innenwandung des Gehäuses (2) definiert und innerhalb des induktiven zylinderförmigen Messvolumens (Ev) angeordnet ist. Damit können durch eine relativ kompakte Anordnung verschiedene Messwerte und Charakteristika des Objekts erfasst werden, um dessen rasche Identifizierung mit hoher Zuverlässigkeit zu gestatten. Über den durch die Beabstandung der Halbspulen gebildeten Spalt ist das längliche Objekt für zumindest die optische Durchmessermessung, für eine allfällige Farbmessung, und eventuell weitere optische Messungen und auch Messungen auf Basis weiterer Messprinzipien zusätzlich zu den induktiven Messungen einfach zugänglich.

Vorzugsweise ist die Länge einer Halbspule zumindest halb so gross wie deren Durchmesser, womit das Gehäuse der Spule bzw. der Halb-Spulen eine optische Abschattung des optischen Messvolumens gegenüber Fremdlicht bewirkt. Damit wird die Messgenauigkeit der Durchmesserbestimmung und Farberkennung verbessert. Auch die Leiterquerschnitts-Messung wird mit zunehmender Länge der Spule genauer, denn je länger eine Spule ist, desto homogener ist deren Magnetfeld über den Querschnitt. Dazu trägt auch das optionale Merkmal bei, dass das induktive zylindrische Messvolumen vorzugsweise durch Kabelbuchsen und weiter vorzugsweise radial eingeschränkt ist. Mit steigender Spulenlänge nimmt also die Abhängigkeit von Messresultat zum momentanen Kabel-Ort innerhalb der Spule ab. Mit zunehmender Länge des induktiven Messvolumens in axialer Richtung des zu identifizierenden Objekts steigt demnach die Messgenauigkeit aller Messsysteme, ob es sich nun um induktive oder rein optische Messungen handelt.

Eine besonders vorteilhafte Ausführungsform einer erfindungsgemässen Anordnung ist weiters dadurch gekennzeichnet, dass der Parallelschwingkreis des induktiven Messsystems mit einer Erregerschaltung verbunden, vorzugsweise mit seiner Eigenfrequenz betrieben und mit einer elektronischen Schaltung zur Spannungsamplitudenmessung verbunden ist.

Eine dazu alternative Ausführungsform sieht vor, dass der Parallelschwingkreis des induktiven Messsystems mit einem Frequenzgenerator einen als induktiven Sensor des induktiven Messsystems genutzten Parallelschwingkreis bilden, der mit einer elektronischen Schaltung zur Messung des Amplitudenganges und/oder des Phasenganges verbunden ist.

Bevorzugt ist das optische scheibenförmige Messvolumen des ersten optischen Messsystems in Längsrichtung mittig und vorzugsweise koaxial im das Objekt umgreifenden induktiven zylinderförmigen Messvolumen positioniert. Damit ist der Bereich der optischen Messung von beiden Seiten her optimal gegenüber Störlicht von aussen abgeschirmt.

Vorteilhafterweise weist das erste optische Messsystem zumindest eine erste Beleuchtungsanordnung mit zumindest einer Lichtquelle und vorzugsweise einer Blende sowie ein jenseits der Halbspulen auf der optischen Hauptachse positioniertes erstes Sensorarray auf. Diese Anordnung erlaubt eine sehr genaue Durchmesserbestimmung des länglichen Objekts bei relativ einfacher konstruktiver Ausführung mit herkömmlichen und bewährten Bauteilen. Überdies kann die Lichtquelle vorteilhafterweise durch den Spalt des Gehäuses, zwischen den Halbspulen und den Kabelbuchsen, das Objekt beleuchten, dessen Schattenwurf ebenso durch diesen Spalt auf das erste Sensorarray fällt und dort detektiert werden kann. Vorzugsweise ist dafür ein Linearsensorarray vorgesehen.

Eine weitere vorteilhafte Ausführungsform der Erfindung ist dadurch gekennzeichnet, dass zumindest ein zweites optisches Messsystem zur Bestimmung der Farbe des Objektes ausgebildet ist. Da Kabeltypen oftmals beispielsweise durch die Farben von deren Ummantelung gekennzeichnet sind, erlaubt eine derartige Sensoranordnung eine rasche und sehr sichere Erkennung des Kabeltyps.

Bevorzugt weist dieses zweite optische Messsystem eine zweite Beleuchtungsanordnung mit mehreren, vorzugsweise drei nahe beieinander platzierten, Lichtquellen mit unterschiedlichen Wellenlängenspektren sowie zumindest ein zweites Sensorarray für das vom Objekt reflektierte Licht auf, wobei dieses in Bezug auf die x-z-Ebene auf der gleichen Seite des scheibenförmigen Messvolumens wie die zweite Beleuchtungsanordnung liegt. Diese Lichtquellen sind vorzugsweise so platziert und ihr Lichtkegel mit einer Blende so geformt, dass ihr Licht nicht auf das Sensorgehäuse sondern nur auf das zu messende Objekt fällt.

Vorzugsweise ist dabei im zweiten optischen Messsystem ein Ablauf implementiert, um die Lichtquellen der zweiten Beleuchtungsanordnung derart anzusteuern, sodass das Objekt sequentiell beleuchtet und somit sequenziell Abbilder in den Wellenlängenspektren der Lichtquellender zweiten Beleuchtungsanordnung auf das zweite Sensorarray projiziert werden. Dieses zweite Sensorarray ist seinerseits mit einer Auswerteeinheit für die bei der Beleuchtung mit der zweiten Beleuchtungsanordnung unterschiedlicher Wellenlängenspektren gemessenen Intensitäten und für die daraus folgende Bestimmung der Farbe des Objekts verbunden.

Eine vorteilhafte Ausführungsform dieser Anordnung ist erfindungsgemäss dadurch gekennzeichnet, dass auf der Hauptachse der optischen Messsysteme ein reflektierendes Langpassfilter angeordnet ist, welches für die Wellenlängenspektren der zweiten Beleuchtungsanordnung reflektierend und für die der ersten durchlässig ist und welches das vom Objekt reflektierte Licht auf das ausserhalb der optischen Hauptachse positionierte und auf das Langpassfilter hin ausgerichtete zweite Sensorarray umlenkt.

Eine weitere vorteilhafte Ausführungsform der Erfindung sieht vor, dass zwischen dem scheibenförmigen Messvolumen und dem Langpassfilter eine Linse angeordnet ist, welche sowohl einmal vom Licht der ersten Beleuchtungsanordnung als Kollimationslinse als auch zweimal als bildgebende Linse vom durch das Objekt reflektierte Licht der zweiten Beleuchtungsanordnung passiert wird. Durch diese quasi-koaxiale Anordnung kann die Kollimationsanordnung der Durchmessermessung auch als bildgebende Linse für die Messanordnung für die Farbe des länglichen Objekts genutzt werden, womit eine einfachere und kompaktere Konstruktion der Gesamtanordnung und eine Einsparung der benötigen Bauteile möglich ist.

Bevorzugt sind gemäss der vorliegenden Erfindung die optischen Messsysteme zur Bestimmung des Durchmessers und der Farbe des Objekts zu einem dritten, virtuellen Messsystem zur Bestimmung der Position des Objekts innerhalb des scheibenförmigen Messvolumens kombiniert. Damit sind die einzelnen optischen Messungen und die induktive Messung unter Einbeziehung der Lageinformationen für das Objekt korrigierbar, um noch genauere Messergebnisse zu erhalten.

Bevorzugt ist dabei die Hauptebene der optischen Messsysteme mit der optischen Hauptachse senkrecht zur Längsachse des induktiven Messvolumens angeordnet.

Eine vorteilhafte erfindungsgemässe Anordnung sieht weiters vor, dass das dritte Messsystem zur Bestimmung der Position eine dritte Beleuchtungsanordnung mit vorzugsweise zwei Lichtquellen, das erste Sensorarray und optional die erste Beleuchtungsanordnung zur triangulatorischen Bestimmung der Position des Objektes (W) innerhalb des Messvolumens (DCPv) umfasst.

Eine alternative Ausführungsform für die zweite Beleuchtungsanordnung ist jene, bei welcher die Lichtquellen der dritten Beleuchtungsanordnung mehrere Lichtquellen mit jeweils unterschiedlichen Wellenlängenspektren aufweisen und ausgelegt sind, um das Objekt sequentiell zu beleuchten und somit sequenziell ein Abbild in den Wellenlängenspektren der Lichtquellen auf das zweite Sensorarray zu projizieren.

Allen Anordnungen ist das vorteilhafte Merkmal der Erfindung gemeinsam, dass die Messebene des zweiten Sensorarrays zur Durchmesserbestimmung senkrecht zur x-y-Ebene verläuft, diese jedoch unter einem kleinen Winkel $\alpha$ zur x-Achse schneidet. Dies ist von besonderer Bedeutung, falls die zwei Orte der Lichtquellen für die Positionsbestimmung auch als Orte für Lichtquellen für die Farbbestimmung verwendet werden.

Eine alternative Ausführungsform kann dadurch gekennzeichnet sein, dass das zweite Sensorarray ein multichromatischer Sensor ist und die Lichtquellen der zweiten Beleuchtungsanordnung gleichzeitig betrieben werden oder durch eine breit- oder mehrbandige Lichtquelle ersetzt sind.

Alternativ oder zusätzlich dazu kann auch vorgesehen sein, dass das zweite Sensorarray ein multichromatischer Sensor ist und zumindest eine der Lichtquellen der zweiten Beleuchtungsanordnung mit einer breit- oder mehrbandigen Lichtquelle ersetzt wird.

Vorteilhafterweise kann die Anordnung auch mit einem Temperatursensor zur Korrektur der temperaturbedingten Messfehler vorgesehen sein.

In diesem Fall ist bevorzugt ein Korrekturablauf im induktiven Messsystem implementiert, um dessen Messwert mit einem Korrekturfaktor als Funktion der Temperatur und mit einem Korrekturfaktor als Funktion des Kabelortes zu versehen.

Um die Kabelerkennung noch aussagekräftiger bzw. eindeutiger zu machen, können bei Bedarf weitere Messsysteme mit von den oben beschriebenen Messprinzipien unterschiedlichen Methoden zusätzlich mit einbezogen werden. So ist es denkbar, die erfindungsgemässe Idee dahingehend auszudehnen, dass ein Messsystem zur Bestimmung des Durchmessers eines elektrischen Leiters innerhalb des Objekts auf Basis einer mechanischen Messung, vorzugsweise einer Messerabstandsmessung zum Zeitpunkt einer Messer-Leiter-Berührung, vorgesehen ist.

Zur Lösung der eingangs gestellten Aufgabe ist auch eine Anlage zur Bearbeitung von länglichen Objekten wie Kabeln, Drähten oder Profilen, mit einer eingangsseitigen Zuführung für die länglichen Objekte koaxial zu deren Längserstreckung vorgesehen. Erfindungsgemäss ist dabei eine Anordnung zur automatischen Erkennung wie in den vorhergehenden Absätzen beschrieben vorgesehen, deren Messvolumen sich vorzugsweise koaxial zur Zuführung der Anlage für die länglichen Objekte erstreckt.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung beschrieben sind. Dabei können die in den Ansprüchen und in der Beschreibung erwähnten Merkmale jeweils einzeln für sich oder in beliebiger Kombination erfindungswesentlich sein.

Der technische Inhalt der Patentansprüche und Figuren ist Bestandteil der Offenbarung. Die Figuren werden zusammenhängend und übergreifend beschrieben. Gleiche Bezugzeichen bedeuten gleiche Bauteile, gleiche Bezugszeichen mit unterschiedlichen Indices geben funktionsgleiche oder ähnliche Bauteile an.

Es zeigt

Fig. 1 einen Längsschnitt in der x-y-Ebene durch eine beispielhafte erfindungsgemässe Sensoranordnung für eine Kabelbearbeitungsmaschine,

Fig. 2 ein Schema der Messsysteme der Anordnung der Fig. 1,

Fig. 3 eine schematische Ansicht in der y-z-Ebene der Messsysteme gemäss Fig. 1,

Fig. 4 ein vereinfachtes elektrisches Schema eines Wirbelstromsensors,

Fig. 5 die Entstehung des Wirbelstromflusses im Kabelquerschnitt,

Fig. 6      einen Schnitt in der x-y-Ebene durch einen erfindungsgemässen Wirbelstromsensor,

Fig. 7      einen Schnitt in der x-z-Ebene durch einen erfindungsgemässen Wirbelstromsensor,

Fig. 8      schematisch das Funktionsschema einer erfindungsgemässen Sensoranordnung für die Durchmesserbestimmung eines Kabels,

Fig. 9      einen Längsschnitt in der y-z-Ebene durch eine erfindungsgemässe Sensoranordnung entlang der optischen Achse,

Fig. 10     einen weiteren Längsschnitt in der y-z-Ebene durch die Sensoranordnung,

Fig. 11     die optischen Verhältnisse einer Sensoranordnung mit einem Doppellinsensystem,

Fig. 12     die optischen Verhältnisse einer Sensoranordnung mit einem Doppellinsensystem mit Spiegel,

Fig. 13     ein Diagramm mit Bildweite, Gegenstandsweite und Abbildungsmassstab als Funktion der Objekt-Bild-Distanz

Fig. 14     ein Diagramm zum Weissabgleich einer Sensoranordnung, und

Fig. 15     ein Diagramm mit den Messwerten eines orangefarbigen Kabels.

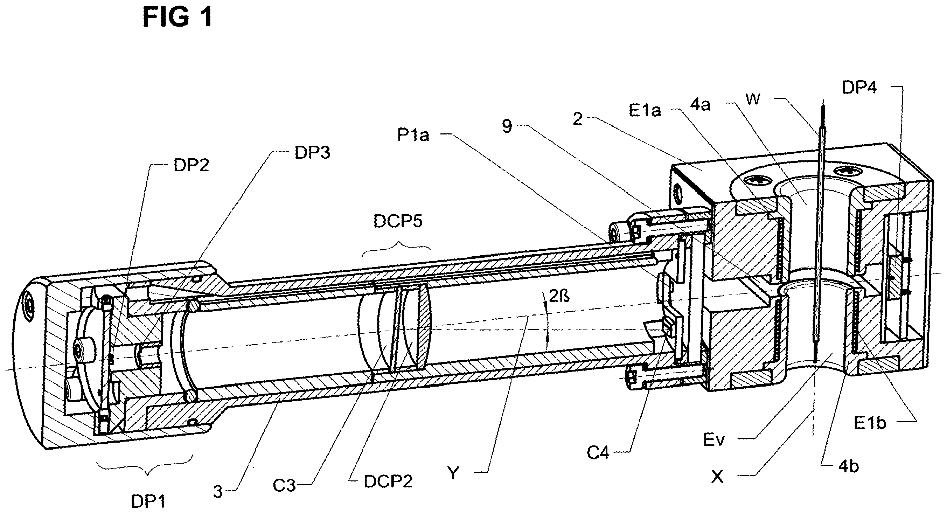

Die Fig. 1 zeigt ein Ausführungsbeispiel einer Sensoranordnung, wie sie vorzugsweise vor oder am Eingang einer Bearbeitungsmaschine für längliche Objekte, insbesondere für Bearbeitungsmaschinen für Kabel od. dgl., positioniert ist, um eine zuverlässige Identifikation des zu bearbeitenden Kabels als Messobjekt W zu ermöglichen. An der Bearbeitungsmaschine sind ein Gehäuse 2 sowie ein Rohr 3 mit einem Teil des optischen Systems der Sensoranordnung befestigt. Durch die Kabelbuchsen 4a, 4b im Gehäuse 2 wird das längliche Objekt W in axialer Richtung hindurchgeführt.

Während der Hindurchbewegung des Objekts W oder auch während einer Stillstandszeit wird mit einem ersten Messsystem E eine elektromagnetische Charakteristik gemessen, woraus vorzugsweise weiter der Querschnitt des leitenden Anteils des Objekts W, insbesondere der Querschnitt des oder der Leiter eines Kabels ermittelt werden kann. Kurz davor, danach oder auch zumindest teilweise gleichzeitig wird mit dem optischen Messsystem D der Durchmesser und optional mit dem optischen Messsystem C die Farbe und optional mit dem Messsystem P die Position des Objektes innerhalb der Kabelbuchse bestimmt. Die Sensoren zur Ermittlung dieser Messgrössen basieren erfindungsgemäss auf unterschiedlichen Messprinzipien, nutzen aber zumindest zum Teil gemeinsame Bereiche oder Elemente der Sensoranordnung.

Die Fig. 1 zeigt einen Schnitt durch die Sensoranordnung in der Ebene, die durch die optische Achse y und die Achse x der Kabelbuchsen 4a und 4b definiert ist. Am äussersten Ende des Rohres 3 ist eine erste Beleuchtungsanordnung DP1 des ersten optischen Messsystems D für die Durchmesserbestimmung mit seiner Lichtquelle DP2 und der Blende DP3 positioniert. Das erste optische Messsystem D kann auch Teil eines weiteren optischen Messsystems P zur Positionsbestimmung des Objekts W sein. Zwischen dem Objekt W und dieser ersten Beleuchtungsanordnung DP1 ist eine abbildende Optik DCP5 eingebaut, deren Langpassfilter C3, welches für sichtbares Licht als Spiegel wirkt, das vom Objekt W reflektierte Licht reflektiert und auf ein zweites Sensorarray C4 eines zweiten optischen Messsystems C für die Farberkennung des Objekts W ausgerichtet ist.

Zwischen den Kabelbuchsen 4a und 4b und dem Gehäuse 2 sind die Halbspulen E1a, E1b für einen Wirbelstromsensor als bevorzugte Variante eines induktiven Messsystems E für die Bestimmung des Querschnitts des Leiters - als Beispiel für eine elektromagnetische Charakteristik - des Objekts W untergebracht. Vorzugsweise ist die Gesamtlänge beider Spulen E1a, E1b zusammen zumindest gleich gross ist wie deren Durchmesser.

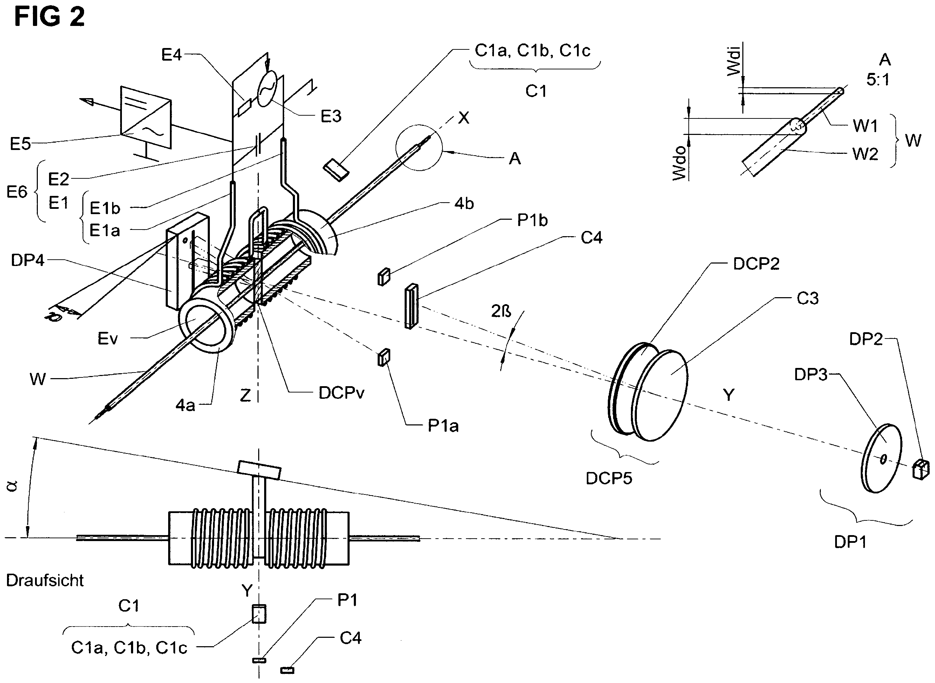

Wie in der Fig. 2 zu sehen ist, definiert die Kabelführungsöffnung der Kabelbuchsen 4a, 4b innerhalb der durch die beiden Halbspulen E1a, E1b gebildeten Spule E1, das induktive zylinderförmige Messvolumen Ev, wobei für den Fall von Spulen E1a, E1b und/oder Kabelbuchsen 4a, 4b mit abweichender Umfangsform auch abweichende geometrische Formen für das Messvolumen Ev vorkommen können. Ausserdem beherbergt das Gehäuse 2 einen Detektor für das optische Messsystem D für die Durchmesserbestimmung, insbesondere ein erstes Sensorarray DP4. Bevorzugt ist dieses erste Sensorarray DP4 als Linearsensorarray ausgeführt.

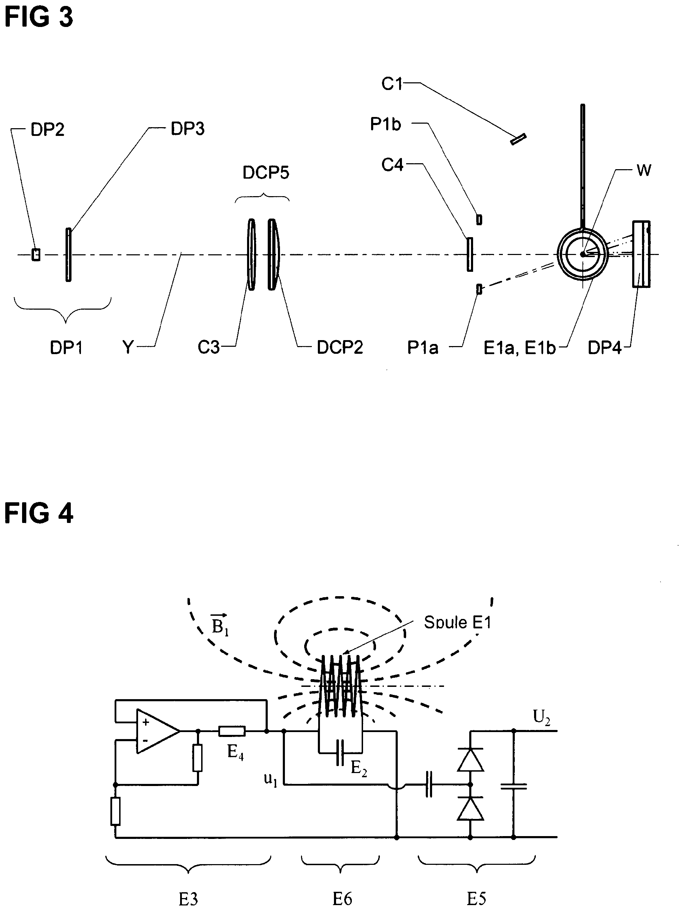

In den Fig. 2 und 3 sind die wesentlichen Elemente der Messsysteme E, D, C, P nochmals schematisch und in ihrer vorteilhaften gegenseitigen Anordnung dargestellt und werden nachfolgend näher erläutert.

Der Wirbelstromsensor als induktives Messsystem E umfasst die beiden zu einer gesamten Spule E1 in Serie geschalteten und gleichsinnig orientierten sowie koaxialen Halbspulen E1a, E1b, die mit der parallel dazu geschalteten Kapazität E2 einen Parallelschwingkreis E6 bilden. Dieser Schwingkreis E6 wird über die Erregerschaltung E3 angeregt und als induktiver Sensor des induktiven Messsystems E genutzt, der vorzugsweise mit seiner Eigenfrequenz betrieben und mit einer elektronischen Schaltung E5 zur Spannungsamplitudenmessung verbunden ist. Eine alternative Ausführungsform sieht anstelle der Erregerschaltung E3 einen Frequenzgenerator vor. In diesem Fall ist die elektronische Schaltung E5 zur Messung des Amplitudenganges und/oder des Phasenganges ausgelegt. Die Erregerschaltung E3 kann auch einen separaten Ausgangswiderstand E4 aufweisen oder kann derart aufgebaut sein, dass die Wirkung eines Ausgangswiderstandes realisiert ist. Der Parallelschwingkreis E6 ist, wie in Fig. 4 vereinfacht und schematisch dargestellt ist, zumindest mit einer Erregerschaltung E3 als auch einer Gleichrichterschaltung E5 verbunden.

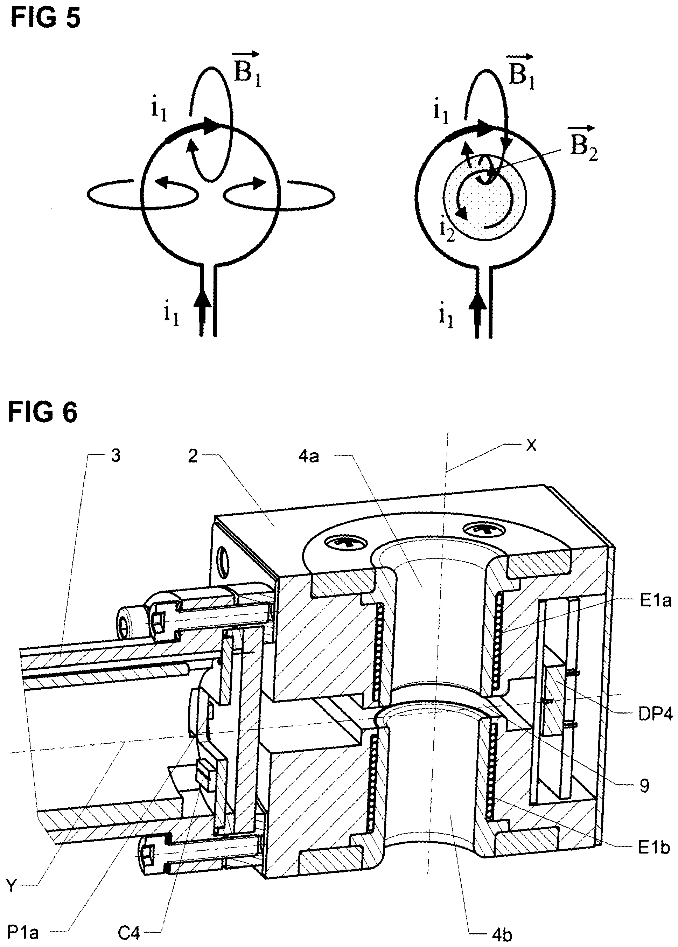

Fig. 5 zeigt die Entstehung des Wirbelstromflusses im Kabelquerschnitt: Wird in das oszillierende Magnetfeld B1 der Spule E1 - hier durch eine einzige Windung symbolisiert - ein elektrischer Leiter W1 - hier der Leiter des Objekts W - gebracht, wird in diesem Leiter W1 ein Wirbelstrom i2 erzeugt, der wiederum ein Magnetfeld B2 erzeugt, welches dem primären Magnetfeld B1 entgegengesetzt ist und versucht, dieses im elektrischen Leiter W1 aufzuheben. Zusammen mit dem ohmschen Widerstand des Leiters W1 bewirkt der Stromfluss i2 Leistungsverluste, welche eine dämpfende Wirkung auf den LC-Schwingkreis E6 haben. Diese Dämpfung ist von der Form, Art, Grösse und Temperatur des elektrischen Leiters W1, aber auch von der Frequenz des magnetischen Wechselfeldes abhängig. Wird der LC-Schwingkreis E6 durch einen elektrischen Leiter W1 in der Spule E1 gedämpft, ist diese Dämpfung als Verminderung der gleichgerichteten Schwingkreisspannung U2 messbar.

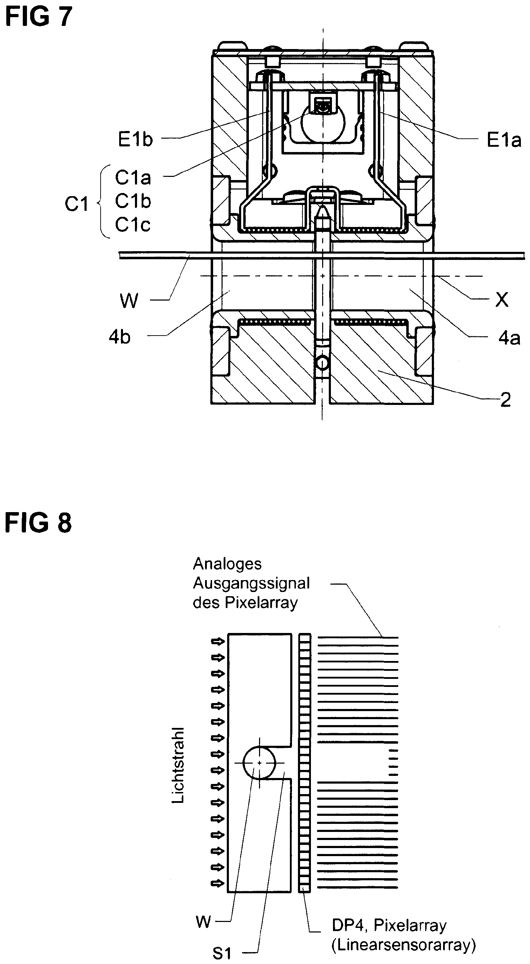

Fig. 6 und Fig. 7 zeigen in vergrössertem Massstab orthogonale Schnitte durch das Gehäuse 2 und die Spulenanordnung E1a, E1b des Wirbelstromsensors des induktiven Messsystems E. Die beiden Kabelbuchsen 4a, 4b mit den Halbspulen E1a und E1b sind in axialer Richtung geringfügig voneinander beabstandet, wodurch sich zwischen diesen beiden Bauteilen ein Spalt 9 ergibt, der im Gehäuse 2 fortgesetzt ist und der Zugang zum Objekt W im Inneren der Kabelbuchsen 4a, 4b für die optischen Messsysteme D, C, P ermöglicht. Das gemeinsame Teilvolumen, das sich durch Spalt 9 und die Kabelführungsöffnung ergibt, bildet das scheibenförmige optische Messvolumen DCPv. Weiters geht aus der Fig. 2 auch hervor, dass vorzugsweise die Hauptebene y-z der optischen Messsysteme D, C, P, in welcher auch die optische Hauptachse y liegt, senkrecht zur Längsachse x des induktiven Messvolumens Ev orientiert ist.

Je schmäler der Spulenspalt 9 im Verhältnis zum Durchmesser der Spulen E1a, E1b ist, desto weniger wird die Magnetfeldhomogenität beeinflusst und je länger die beiden Spulen E1a, E1b sind, desto homogener ist das Magnetfeld über den Spulenquerschnitt verteilt. Die Magnetfeldhomogenität ist für eine lageunabhängige Leiterquerschnittsmessung wichtig. Ebenso wie das minimale Spulenlängen zu Durchmesser-Verhältnis haben die Kabelbuchsen 4a, 4b die Aufgabe, das magnetische Feld im Messvolumen Ev möglichst homogen zu halten, indem sie die windungsnahe Region des Spulenquerschnittes mit erhöhter magnetischer Flussdichte vom Messvolumen Ev ausschliessen.

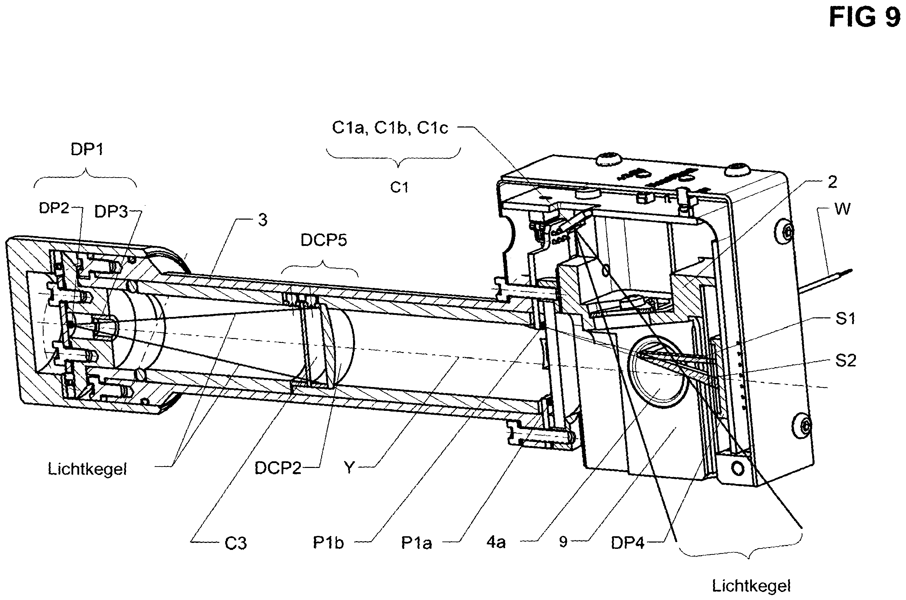

Zur optischen Messung des Aussendurchmessers des Objekts W wird dieses innerhalb der Kabelbuchse 4a, 4b im Bereich des optischen Messvolumens DCPv durch den Spalt 9 hindurch beleuchtet. Wie in Fig. 9 deutlich dargestellt ist, wird das Licht der ersten Beleuchtungsanordnung DP1 durch die Optik DCP5 des ersten optischen Messsystems D kollimiert, trifft auf das Objekt W und ruft einen durch S1 symbolisierten Schattenwurf hervor. Wie in Fig. 8 schematisch dargestellt ist, ruft dieser Schattenwurf S1 auf dem ersten Sensorarray DP4 für jedes Pixel unterschiedliche Spannungs-Pegel hervor, aus deren Verteilung auf den Kabeldurchmesser rückgeschlossen werden kann. Vorteilhafterweise verläuft die Messebene des ersten Sensorarrays DP4, insbesondere in der vorteilhaften Ausführung als Linearsensorarray, senkrecht zur x-y-Ebene, kann diese jedoch unter einem kleinen Winkel $\alpha$ zur x-Achse schneiden (siehe Fig. 2).

Die Parallelität des Lichtes und die Pixelweite des ersten Sensorarrays DP4 sind massgebend für die Genauigkeit der Messung. Das Licht der ersten Beleuchtungseinrichtung DP1 mit Lichtquelle DP2, bevorzugt als Infrarot-LED ausgeführt, wird durch die Linse DCP2 kollimiert, während es durch den Langpassfilter C3 unverändert hindurchtritt. Für andere Wellenlängen, insbesondere im Wellenlängenbereich des sichtbaren Lichts, verhält sich das Langpassfilter C3 aber wie ein Spiegel.

Das scheibenförmige optische Messvolumen DCPv wird zur Durchmesserbestimmung und optional zur Erkennung der Farbe des Objekts W verwendet. Das dafür vorgesehene zweite optische Messsystem C umfasst, wie in den Fig. 2 und 3 zu entnehmen ist, eine zweite Beleuchtungsanordnung mit mehreren nahe beieinander platzierten Lichtquellen C1a, C1b, C1c mit unterschiedlichen Wellenlängenspektren. Vorzugsweise sind drei Lichtquellen vorgesehen. Die Lichtquellen sind beispielsweise als farbige LEDs (z.b. RGB-LEDs) ausgeführt.

Dabei ist vorzugsweise im zweiten optischen Messsystem C ein Ablauf implementiert, beispielsweise als ausführbares Programm in einer Steuer- und Auswerteeinheit dieses Messsystems, mit welchem die Lichtquellen C1a, C1b, C1c der zweiten Beleuchtungsanordnung derart angesteuert werden, um das Objekt W sequentiell zu beleuchten und somit sequenziell ein Abbild in den Wellenlängenspektren der Lichtquellen auf ein zweites Sensorarray C4 dieses Messsystems C zu projizieren. In der Auswerteeinheit des optischen Messsystems C für die Farbbestimmung werden die bei der Beleuchtung des Objekts W mit den Lichtquellen unterschiedlicher Wellenlängenspektren gemessenen Intensitäten für die Bestimmung der Farbe des Objekts W genutzt. Das zweite Sensorarray C4 befindet sich - siehe dazu die Fig. 1 und Fig. 2 - in Bezug auf die x-y-Ebene auf der gleichen Seite des scheibenförmigen Messvolumens DCPv wie die zweite Beleuchtungsanordnung C1. Alternativ zum zweiten Sensorarray C4, das für die Wellenlängen aller drei Lichtquellen der zweiten Beleuchtungsanordnung empfindlich ist, könnte ein multichromatischer Sensor, der aus drei für jeweils unterschiedliche Wellenlängen empfindliche Sensoren besteht, vorgesehen sein.

Das auf der Hauptachse y der optischen Messsysteme D, C, P positionierte Langpassfilter C3 ist für die Wellenlängen der Lichtquellen der zweiten Beleuchtungsanordnung C1 reflektierend und lenkt dadurch das vom Objekt W reflektierte Licht auf das ausserhalb der optischen Hauptachse y positionierte zweite Sensorarray C4 um. Das Langpassfilter C3 wird also vom Licht des ersten Beleuchtungsanordnung DP1 durchsetzt, welches dann durch die Linse DCP2 hindurchtritt und dabei kollimiert wird. Vom Licht der zweiten Beleuchtungsanordnung C1 wird die Linse DCP2 aber zweimal passiert - nach Reflexion durch das Objekt W als auch nach Reflexion am Langpassfilter C3 - sodass deren Brechkraft zweimal genutzt und die bildgebende Brennweite fast halbiert wird. Damit das Bild etwas seitlich der optischen Achse y entsteht, sodass das zweite Sensorarray C4 den Strahlengang nicht stört, ist das Lot des im zweiten Messsystem C reflektierenden Langpassfilters C3 in der x-y-Ebene in einem kleinen Winkel β zur optischen Hauptachse y angeordnet.

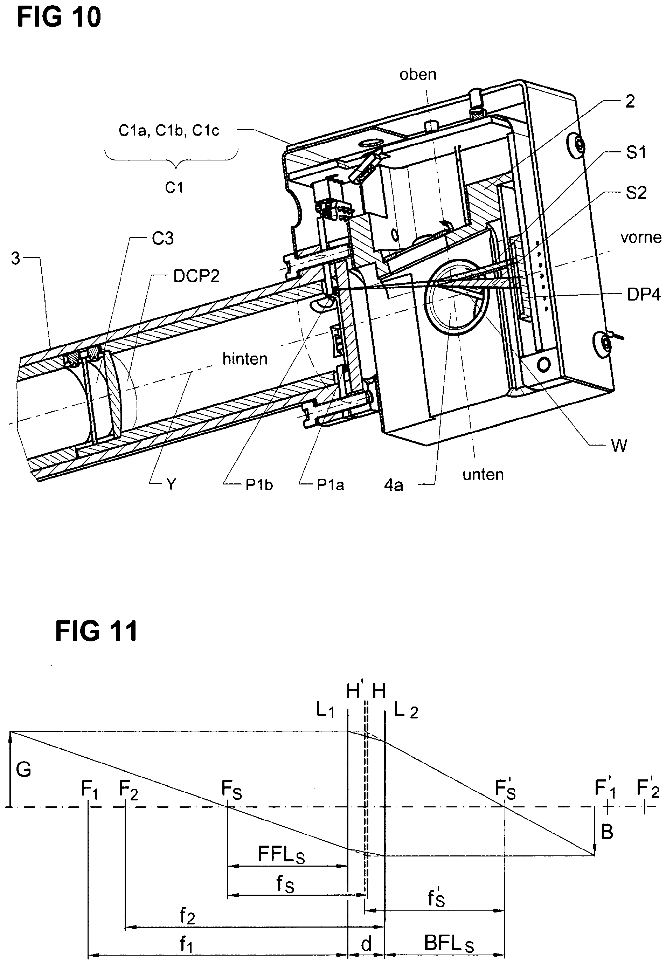

Um die Kabelfarbe zu bestimmen, werden mittels des zweiten Sensorarrays C4 vorzugsweise sequentiell drei Bilder mit jeweils unterschiedlicher Beleuchtung, beispielsweise unter rotem, grünem und blauem Licht, gemacht. Durch die projizierten Farbintensitäten kann dann in der Auswerteeinheit die Farbe des Objekts W berechnet werden. Zu beachten ist dabei, dass die vom zweiten Sensorarray C4 gemessene Farbintensität quadratisch zur Distanz zwischen den Lichtquellen und dem Objekt W sowie quadratisch zur Distanz zwischen Objekt W und dem zweiten Sensorarray C4 abnimmt. Diese Kabel-Ortsabhängigkeit kann zum Beispiel mit angepasster Belichtungszeit korrigiert werden. Wo sich das Kabel W innerhalb des optischen Messvolumens DCPv befindet, kann mit einer einfachen Triangulation mit zwei Schatten-Kanten berechnet werden, wie in Fig. 10 dargestellt ist. Mit Hilfe der Kabelposition kann eine Korrektur der Ausgangssignale des zweiten Sensorarrays C4 für die jeweilige Wellenlänge des Objekts W durch Interpolation zwischen im Messvolumens DCPv empirisch ermittelten Intensitätskorrekturwerten erfolgen.

Für die ortsabhängige Messwertkompensation ist vorzugsweise das erste optischen Messsystem D für die Durchmessermessung mit zwei weiteren Lichtquellen P1a und P1b der dritten Beleuchtungsanordnung P1 zu einem dritten, virtuellen optischen Messsystem P zur Ermittlung der Position des Objekts W innerhalb des scheibenförmigen Messvolumens DCPv kombiniert. Dieses dritte optische Messsystem P nutzt mindestens die zwei Lichtquellen P1a, P1b der dritten Beleuchtungsanordnung P1oder die erste Beleuchtungsanordnung DP1 zusammen mit einer Lichtquelle der dritten Beleuchtungsanordnung P1, sowie das erste Sensorarray DP4.

Alternativ kann auch eine der Lichtquellen, insbesondere der Lichtquelle des ersten Beleuchtungsanordnung DP1 bei allein vorhandener Durchmessermessung, mit einer zusätzlichen Lichtquelle kombiniert werden. Wichtig ist dabei nur eine Beabstandung der verwendeten Lichtquellen in Umfangsrichtung der Kabelbuchsen 4a, 4b bzw. des scheibenförmigen optischen Messvolumens DCPv. Diese beiden Lichtquellen ergeben winkelmässig unterschiedliche Schattenwürfe S1, S2, deren Abstand mit Hilfe des ersten Sensorarrays DP4 bestimmt und aufgrund der bekannten geometrischen Verhältnisse in eine Ortsinformation für das Objekt W im Inneren der Kabelbuchsen 4a, 4b bzw. des induktiven Messvolumens Ev als auch des optischen Messvolumens DCPv umgerechnet werden kann.

Nachfolgend wird ein konkretes Konstruktionsbeispiel der geometrischen Optik einer Ausführungsform einer erfindungsgemässen Sensoranordnung zur Kabelfarb-Erkennung vorgestellt.

Fig. 11 zeigt ein Doppellinsensystem mit zwei gleichen Linsen $L_1$ und $L_2$, ihre zugehörigen Brennpunkte $F_1$ und $F_2$, ihre jeweiligen Brennweiten $f_1$ und $f_2$, den abzubildenden Gegenstand G und das Bild B. Die Linsen $L_1$ und $L_2$ seien im Abstand d voneinander entfernt. Um den Strahlengang und die optische Berechnungen zu vereinfachen, kann das Doppellinsensystem durch eine einzige Linse mit ihren Hauptebenen H und H' und ihren zugehörigen System-Brennpunkten Fs und Fs' ersetzt werden. Der Abstand von Fs zu $L_1$ wird auch $FFL_S$ (front focal length) genannt und der Abstand von $F_S$' zu $L_2$ $BFL_S$ (back focal length).

Dabei gilt:

$$\frac{1}{FFL_S} = \frac{1}{f_1} + \frac{1}{f_2 - d} \qquad \textbf{1)}$$

$$FFL_S = \frac{f_1(f_2 - d)}{(f_1 + f_2) - d} \qquad \textbf{2)}$$

$$\frac{1}{BFL_S} = \frac{1}{f_2} + \frac{1}{f_1 - d} \qquad \textbf{3)}$$

$$BFL_S = \frac{f_2(f_1 - d)}{(f_1 + f_2) - d} \qquad \textbf{4)}$$

$$\frac{1}{f_S} = \frac{1}{f_1} + \frac{1}{f_2} - \frac{d}{f_1 f_2} \qquad \textbf{5)}$$

$$f_S = \frac{1}{\frac{1}{f_1} + \frac{1}{f_2} - \frac{d}{f_1 f_2}} = \frac{f_1 f_2}{f_1 + f_2 - d} \qquad \textbf{6)}$$

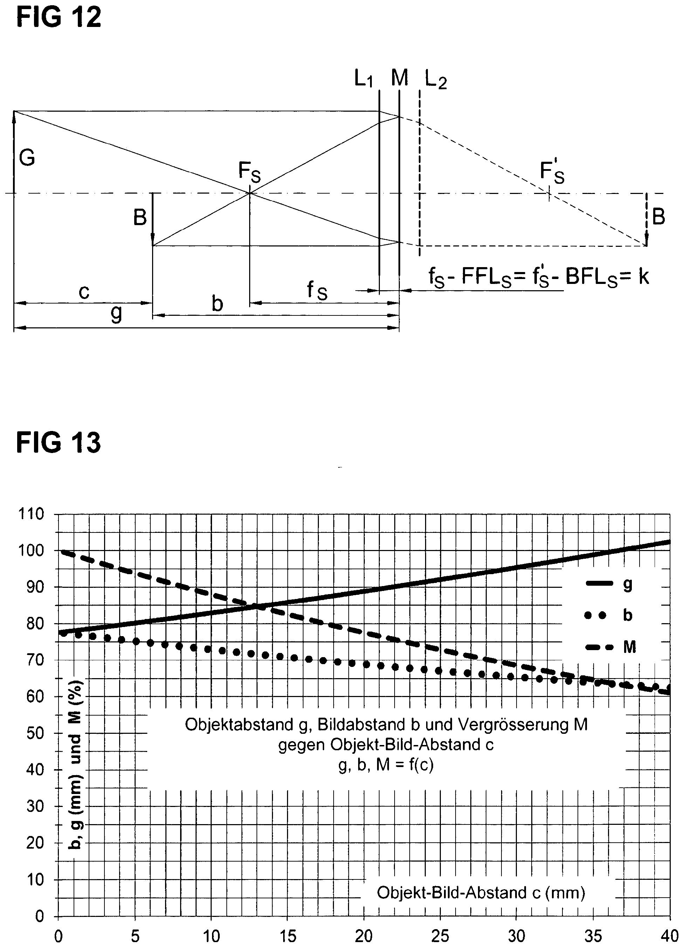

Fig. 12 zeigt das Doppellinsensystem, wenn in der Hauptebene H ein Spiegel M platziert wird. Dieser bewirkt, dass Bild B auf die Objektseite projiziert und $L_2$ weggelassen werden kann, da $L_1$ zusätzlich auch die Funktion von $L_2$ übernimmt. Fig. 12 zeigt somit schematisch die optische Situation im oben erläuterten optischen Messsystem C für die Kabelfarbe, wie sie am deutlichsten in den Fig. 2 und 3 dargestellt ist. Das reflektierende Langpassfilter C3 entspricht dem Spiegel M und die Linsen $L_1$ bzw. $L_2$ entsprechen der Kollimationslinse DCP2.

Mit dem keilförmigen Rohrstück der Optik DCP5 in Fig. 1 zwischen Spiegel M und Linse $L_1$ ist die Distanz k = 6.575 mm zwischen M und $L_1$ gegeben. Anhand der Fig. 11 und Fig. 12 kann der Abstand d zwischen den Linsen $L_1$ und $L_2$ wie folgt berechnet werden:

$$k = f_S(d) - FFL_S(d) \qquad \textbf{7)}$$

$$k = \frac{f_1 f_2}{f_1 + f_2 - d} - \frac{f_1(f_2 - d)}{(f_1 + f_2) - d} \qquad \textbf{8)}$$

$$k = \frac{f^2}{2f - d} - \frac{f^2 - fd}{2f - d} \qquad \textbf{9)}$$

$$(2f - d)k = fd \qquad \textbf{10)}$$

$$fd + kd = 2fk \qquad \textbf{11)}$$

$$d = \frac{2fk}{f + k} \qquad \textbf{12)}$$

$$d = \frac{2 \cdot 71 \cdot 6.575}{71 + 6.575} = 12.035 \qquad \textbf{13)}$$

Unter Verwendung von Formel 6 und für f = 71 mm soll nun die System-Brennweite fs berechnet werden

$$f_S = \frac{71 \cdot 71}{71 + 71 - 12.035} = 38.787 \qquad \textbf{14)}$$

In der Konstruktion (Fig. 1, Fig. 2 und Fig. 3) ergab sich ein c (Distanz von Gegenstand G zum Bild B also von der Längsachse x zum zweiten Sensorarray C4) von 33.5 mm. Nun stellt sich die Frage, wie gross die Distanzen b (Sensor - Spiegel) und g (Längsachse x - Spiegel C3) sein müssen, damit sich beim zweiten Sensorarray C4 ein scharfes Bild ergibt.

Dies ergibt sich wie folgt (mit c = 33.5 mm):

$$\frac{1}{f_s} = \frac{1}{g} + \frac{1}{b} \qquad\qquad \textbf{15)}$$

$$g = c + b \qquad\qquad \textbf{16)}$$

$$\frac{1}{f_s} = \frac{1}{c+b} + \frac{1}{b} \qquad\qquad \textbf{17)}$$

$$\frac{1}{f_s} - \frac{1}{b} = \frac{1}{c+b}$$

$$\frac{b - f_s}{f_s b} = \frac{1}{c+b}$$

$$(b - f_s)(c + b) = f_s b$$

$$bc + b^2 - f_s c - f_s b = f_s b$$

$$b^2 + bc - 2f_s b - f_s c = 0$$

$$b^2 + b(c - 2f_s) - f_s c = 0$$

$$b = \frac{(2f_s - c)}{2} \pm \sqrt{\frac{(c - 2f_s)^2}{4} + f_s c} \qquad\qquad \textbf{18)}$$

$$b = \frac{(2 \cdot 38.787 - 33.5)}{2} \qquad\qquad \textbf{19)}$$

$$\pm \sqrt{\frac{(33.5 - 2 \cdot 38.787)^2}{4} + 38.787 \cdot 33.5}$$

$$b = 22.037 \pm 42.249 = 64.286$$

$$g = 33.5 + 64.286 = 97.786 \qquad\qquad \textbf{20)}$$

Da nun alle Dimensionen gegeben sind, kann noch der Abbildungsmassstab M berechnet werden.

$$M = \frac{b}{g} = \frac{B}{G} \qquad\qquad \textbf{21)}$$

$$M = \frac{64.286}{97.786} = 0.657 \qquad\qquad \textbf{22)}$$

Ist das zweite Sensorarray C4 ein Linearsensorarray mit einer Auflösung von 400 dpi und 128 Pixel ergibt sich eine aktive Sensor-Array-Länge $l_{SA}$:

$$l_{SA} = \frac{25.4 \cdot 128}{400} = 8.13 \, mm \qquad \qquad \textbf{23)}$$

Somit ergibt sich eine maximale abzubildende Gegenstandsgrösse von:

$$G = \frac{B}{M} = \frac{l_{SA}}{M} = \frac{8.128}{0.657} = 12.37 \, mm \qquad \qquad \textbf{24)}$$

Mit der System-Brennweite $f_S$ der Linse aus Formel 14 können, wie in Fig. 13 dargestellt, die Bildweite b und die Gegenstandsweite g in Abhängigkeit der Gegenstand-Bild-Distanz c also der Längsachse x - zweites Sensorarray C4 - Distanz für das konkrete Ausführungsbeispiel unter Verwendung der Gleichung 18 graphisch dargestellt werden.

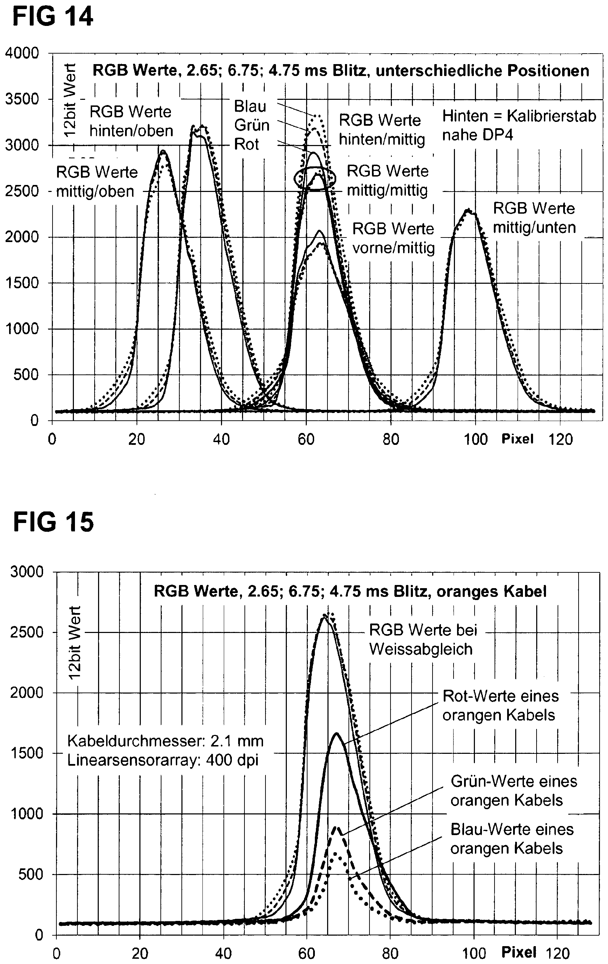

Wenn die Gegenstand-Sensor-Distanz c gegen 0 geht, geht der Abbildungsmassstab, wie in Fig. 14 zu erkennen ist, gegen 100 %. Das ist die bekannte 1/1 Abbildung mit g = b= 2*$f_S$.

Bevor mit dem zweiten Sensorarray C4 die Kabelfarbe gemessen werden kann, muss ein Weissabgleich vorgenommen werden. Dazu wird ein weisser Kalibrationsstab derart in die Kabelbuchsen 4a, 4b gelegt, dass er möglichst nahe bei der zweiten Beleuchtungsanordnung C1 liegt, damit das zweite Sensorarray C4 die grösste Helligkeit misst. Die maximal erlaubten Beleuchtungszeiten bei der Beleuchtung mit den unterschiedlichen Wellenlängen (rot, grün, blau) werden dann so eingestellt, dass die gemessenen Amplituden etwa 90% des Messbereiches ausmachen. Danach wird der Kalibrationsstab in die Längsachse x gelegt und die Belichtungszeiten so eingestellt, dass die vom zweiten Sensorarray C4 gemessenen RGB-Integralwerte alle gleich gross sind. Dabei werden die zwei höheren Integralwerte dem tiefsten angepasst, sodass die zuvor bestimmten maximalen Belichtungszeiten von keiner Farbe überschritten werden (siehe Fig. 14). Da die Helligkeit des Bildes mit zunehmender Distanz zwischen Lichtquelle und Objekt W und zwischen Objekt W und Linse DCP2 abnimmt, müssen die Helligkeitswerte für die unterschiedlichen Wellenlängen je nach Kabelposition gewichtet werden. In Fig. 14 sind zum Beispiel für sechs Kabelpositionen innerhalb der Kabelbuchsen 4a, 4b RGB-Messwerte dargestellt: Für den weissen Kalibrationsstab im Zentrum der Kabelbuchsen 4a, 4b gelten die mit "centric/centric" bezeichneten Kurven. Befindet sich der Kalibrationsstab in der Kabelbuchsen 4a, 4b nahe dem zweiten Sensorarray C4 gelten die die mit "back/centric" bezeichneten Linien. In Fig. 10 sind die Ortsangaben back, front, bottom und top gekennzeichnet damit die in Fig. 10 dargestellten Messwerte richtig interpretiert werden können.

Das Verhältnis der mit einem Kalibrationsstab gemessenen RGB-Integralwerte eines bestimmten Ortes zu den RGB-Integralwerten des Zentrums sind Farbkorrekturwerte. Sie Sind ortsabhängig. Fig. 15 schliesslich zeigt RGB-Messwerte eines orangefarbigen Kabels als Objekt W im Zentrum der Kabelbuchsen.

Nebst den schon erwähnten Vorteilen der quasi-koaxialen Anordnung der optischen Messsysteme D, C und P, ob real oder virtuell, wie der kompakten, robusten, mechanischen und funktionell synergienutzenden Bauweise besteht ein weiterer Vorteil darin, dass viele Schaltungsteile der Elektronik wie Kommunikationsschnittstelle, Mikrokontroller, Speisung, LED-Anzeigen und Schutzbeschaltungen für alle drei Sensoren verwendet werden können.

Je grösser die Brennweite der Linse DCP2 für die Kollimation ist, desto besser ist die Kollimation, d.h. die Parallelität des Lichtes und desto scharfkantiger ist der Schattenwurf für die Durchmessermessung. Auch für die Farbbestimmung ist eine grosse Brennweite der Linse DCP2 vorteilhaft, da dann die Kabel-Linsen-Distanz g grösser wird und somit die Bildschärfe für die Farberkennung erhalten bleibt, auch wenn das Objekt W nicht zentrisch durch die Kabelbuchsen 4a, 4b laufen sollte. Mit zunehmender Brennweite nimmt jedoch für die Durchmessermessung wie auch für die Farbbestimmung die Lichtintensität ab, was mit einer längeren Belichtungszeit kompensiert werden muss, sodass für beide Funktionalitäten ein Kompromiss zwischen Lichtintensität und Bildschärfe gefunden werden muss.

Die oben erläuterte Anordnung zur automatischen Erkennung von länglichen Objekten mit ihrem induktiven Messsystem E und den rein optischen Messsystemen D, C, P kann bei Bedarf mit weiteren Messsystemen verknüpft werden. Bei Kabelbearbeitungsanlagen sind oftmals Vorrichtungen zum Ablängen oder Abisolieren der Kabel vorhanden, die gegeneinander bewegbare Klemmbacken und Messer umfassen. Für derartige Anlagen können die Messsysteme E, D, C, P mit zumindest einem weiteren Messsystem kombiniert werden, welches zur Bestimmung des Aussendurchmessers des Objekts mittels einer Klemmbackenabstandsmessung oder welches zur Bestimmung des Durchmessers eines elektrischen Leiters innerhalb des Objekts auf Basis einer Messerabstandsmessung zum Zeitpunkt einer Messer-Leiter-Berührung vorgesehen ist. Messanordnungen auf elektrischer Basis, insbesondere durch Überwachung von kapazitiven und/oder induktiven Charakteristika, sind hinlänglich bekannt.

Um temperaturbedingte Messfehler kompensieren zu können, ist die Verwendung eines Temperatursensors in Verbindung mit den Messsystemen E, D, C, P vorteilhaft. Dabei ist für eine automatisierte Kompensation bevorzugt ein Korrekturablauf im jeweiligen Messsystem implementiert, um dessen Messwert mit einem Korrekturfaktor als Funktion der Temperatur und mit einem Korrekturfaktor als Funktion des Kabelortes zu versehen.

**Patentansprüche**

1. Anordnung zur automatischen, berührungslosen Erkennung von länglichen Objekten (W) wie Kabeln, Drähten oder Profilen, mit zumindest einem induktiven Messsystem (E) zur Bestimmung einer elektromagnetischen Charakteristik des Objektes (W) und zumindest einem ersten optischen Messsystem (D) für das Objekt (W) in einem gemeinsamen Gehäuse (2), wobei zumindest ein erstes optisches Messsystem (D) zur Bestimmung des Aussendurchmessers (Wdo) des Objektes (W) ausgebildet ist, wobei ein optisches scheibenförmiges Messvolumen (DCPv) definiert ist, **dadurch gekennzeichnet, dass** das induktive Messsystem (E) als Wirbelstromsensor zur Bestimmung einer elektromagnetischen Charakteristik des Objektes (W) ausgelegt ist und zwei in Serie geschaltete, gleichsinnig orientierte und koaxiale Halbspulen (Ela, E1b) aufweist, die in axialer Richtung einen Abstand voneinander aufweisen, koaxial zur Längsachse (x) das Objekt (W) umwinden, so dass deren Innenraum ein induktives zylinderförmiges Messvolumen (Ev) definiert, wobei die Halbspulen (Ela, E1b) zusammen mit einer elektrisch parallel dazu geschalteten Kapazität (E2) einen als induktiven Sensor des induktiven Messsystems (E) genutzten Parallelschwingkreis (E6) bilden, der mit einer elektronischen Auswerteschaltung (E5) verbunden ist, **und dass** das optische scheibenförmige Messvolumen (DCPv) durch den Abstand der Halbspulen (E1a, E1b) und die Innenwandung des Gehäuses (2) definiert und innerhalb des induktiven zylinderförmigen Messvolumens (Ev) angeordnet ist.

2. Anordnung nach Anspruch 1, **dadurch gekennzeichnet, dass** die Länge einer Halbspule (E1a, E1b) zumindest halb so gross ist wie deren Durchmesser **und dass** das induktive zylindrische Messvolumen (Ev) vorzugsweise durch Kabelbuchsen (4a, 4b) und vorzugsweise radial eingeschränkt ist.

3. Anordnung nach einem der Ansprüche 1 oder 2, **dadurch gekennzeichnet, dass** der Parallelschwingkreis (E6) des induktiven Messsystems (E) mit einer Erregerschaltung (E3) verbunden, vorzugsweise mit seiner Eigenfrequenz betrieben, und mit einer elektronischen Schaltung (E5) zur Spannungsamplitudenmessung verbunden ist, oder **dass** der Parallelschwingkreis (E6) des induktiven Messsystems (E) mit einem Frequenzgenerator und mit einer elektronischen Schaltung (E5) zur Messung des Amplitudenganges und/oder des Phasenganges verbunden ist.

4. Anordnung nach einem der Ansprüche 1 bis 3, **dadurch gekennzeichnet, dass** das optische scheibenförmige Messvolumen (DCPv) des ersten optischen Messsystems (D) vorzugsweise in Längsrichtung mittig und vorzugsweise koaxial im das Objekt (W) umgreifenden induktiven zylinderförmigen Messvolumen (Ev) positioniert ist.

5. Anordnung nach einem der Ansprüche 1 bis 4, **dadurch gekennzeichnet, dass** das erste optische Messsystem (D) zumindest eine erste Beleuchtungsanordnung (DP1) mit zumindest einer ersten Lichtquelle (DP2) und vorzugsweise eine Blende (DP3) sowie ein jenseits der Halbspulen (E1a, E1b) auf der Hauptachse (y) positioniertes erstes Sensorarray (DP4) aufweist und vorzugsweise zumindest ein zweites optisches Messsystem (C) zur Bestimmung der Farbe des Objektes (W) ausgebildet ist.

6. Anordnung nach Anspruch 5, **dadurch gekennzeichnet, dass** das zweite optische Messsystem (C) eine zweite Beleuchtungsanordnung (C1) mit mehreren, vorzugsweise drei und auch vorzugsweise nahe beieinander platzierten Lichtquellen (C1a, C1b, C1c) mit unterschiedlichen Wellenlängenspektren sowie zumindest ein zweites Sensorarray (C4) für das vom Objekt (W) reflektierte Licht aufweist, wobei das zweite Sensorarray (C4) in Bezug auf die x-z-Ebene auf der gleichen Seite des scheibenförmigen Messvolumens (DCPv) wie die zweite Beleuchtungsanordnung (C1) liegt.

7. Anordnung nach Anspruch 6, **dadurch gekennzeichnet, dass** im zweiten optischen Messsystem (C) ein Ablauf implementiert ist, die Lichtquellen (C1a, C1b, C1c) der zweiten Beleuchtungsanordnung (C1) derart anzusteuern, um das Objekt (W) sequentiell zu beleuchten und somit sequenziell Abbilder in den Wellenlängenspektren der Lichtquellen der zweiten Beleuchtungsanordnung auf das zweite Sensorarray (C4) zu projizieren, wobei dieses mit einer Auswerteeinheit für die bei der Beleuchtung mit den Lichtquellen unterschiedlicher Wellenlängenspektren gemessenen Intensitäten und für die daraus folgende Bestimmung der Farbe des Objekts (W) verbunden ist.

8. Anordnung nach Anspruch 6 oder 7, **dadurch gekennzeichnet, dass** auf der Hauptachse (y) des ersten und zweiten optischen Messsystems (D, C) ein reflektierendes Langpassfilter (C3) angeordnet ist, welches für die Wellenlängenspektren der zweiten Beleuchtungsanordnung (C1) reflektierend und für jene der ersten Beleuchtungsanordnung (DP1) durchlässig ist und welches das vom Objekt (W) reflektierte Licht auf das ausserhalb der optischen Hauptachse (y) positionierte und auf das Langpassfilter (C3) hin ausgerichtete zweite Sensorarray (C4) umlenkt.

9. Anordnung nach Anspruch 8, **dadurch gekennzeichnet, dass** zwischen dem scheibenförmigen Messvolumen (DCPv) und dem Langpassfilter (C3) eine Linse (DCP2) angeordnet ist, welche sowohl einmal als Kollimationslinse (DCP2) vom Licht der ersten Beleuchtungsanordnung (DP1) als auch zweimal als bildgebende Linse (DCP2) vom durch das Objekt (W) reflektierte Licht der zweiten Beleuchtungsanordnung (C1) passiert wird.

10. Anordnung nach einem der Ansprüche 5 bis 9, **dadurch gekennzeichnet, dass** die optischen Messsysteme (D) zur Bestimmung des Durchmessers und (C) zur Bestimmung der Farbe des Objekts (W) zu einem dritten, virtuellen optischen Messsystem (P) zur Bestimmung der Position des Objekts (W) innerhalb des scheibenförmigen Messvolumens (DCPv) kombiniert sind, wobei insbesondere die Hauptebene (y-z) des ersten, zweiten und dritten optischen Messsystems (D, C, P) mit der optischen Hauptachse (y) senkrecht zur Längsachse (x) des induktiven Messvolumens (Ev) angeordnet ist.

11. Anordnung nach Anspruch 10, **dadurch gekennzeichnet, dass** das dritte Messsystem (P) eine dritte Beleuchtungsanordnung (P1) mit vorzugsweise zwei Lichtquellen (P1a, P1b), das erste Sensorarray (DP4) und optional die erste Beleuchtungsanordnung (DP1) zur triangulatorischen Bestimmung der Position des Objektes (W) innerhalb des Messvolumens (DCPv) umfasst, wobei insbesondere die dritte Beleuchtungsanordnung (P1) mehrere Lichtquellen mit jeweils unterschiedlichen Wellenlängenspektren aufweisen und ausgelegt sind, um das Objekt (W) sequentiell zu beleuchten und somit sequenziell ein Abbild in den Wellenlängenspektren der Lichtquellen auf das zweite Sensorarray (C4) zu projizieren.

12. Anordnung nach einem der Ansprüche 5 bis 11, **dadurch gekennzeichnet, dass** die Messebene des ersten Sensorarrays (DP4) senkrecht zur x-y-Ebene verläuft, diese jedoch unter einem kleinen Winkel ($\alpha$) zur x-Achse schneidet.

13. Anordnung nach einem der Ansprüche 6 bis 12, **dadurch gekennzeichnet, dass** das zweite Sensorarray (C4) ein multichromatischer Sensor ist und die Lichtquellen (C1a, C1b und C1c) der zweiten Beleuchtungsanordnung (C1) gleichzeitig betrieben werden oder durch eine breit- oder mehrbandige zweite Lichtquelle ersetzt werden, und/oder zumindest eine der Lichtquellen der dritten Beleuchtungsanordnung (P1) mit einer breit- oder mehrbandigen Lichtquelle ersetzt wird.

14. Anordnung nach einem der Ansprüche 1 bis 13, **dadurch gekennzeichnet, dass** die Anordnung einen Temperatursensor (T) aufweist.

15. Anordnung nach einem der Ansprüche 1 bis 14, **dadurch gekennzeichnet, dass** ein Korrekturablauf im induktiven Messsystem (E) implementiert ist, um dessen Messwert mit einem Korrekturfaktor als Funktion der Temperatur und mit einem Korrekturfaktor als Funktion der Position des Objekts (W) zu versehen.

16. Anlage zur Bearbeitung von länglichen Objekten (W) wie Kabeln, Drähten oder Profilen, **dadurch gekennzeichnet, dass** die Anlage eine eingangsseitige Zuführung für die länglichen Objekte (W) koaxial zu deren Längserstreckung aufweist, wobei die Anlage eine Anordnung nach einem der Ansprüche 1 bis 15 aufweist.

## Claims

1. Arrangement for automatic, contactless detection of elongated objects (W) such as cables, wires or profiles, having at least one inductive measuring system (E) for determining an electromagnetic characteristic of the object (W) and at least one first optical measuring system (D) for the object (W) in a common housing (2), wherein at least a first optical measuring system (D) is designed to determine the outer diameter (Wdo) of the object (W), wherein an optical disc-shaped measuring volume (DCPv) is defined, **characterized in that** the inductive measuring system (E) is designed as an eddy current sensor for determining an electromagnetic characteristic of the object (W) and includes two series-connected, co-directionally oriented and coaxial half-coils (E1a, E1b), which are located at a distance from each other in the axial direction and encircle the object (W) coaxially with respect to the longitudinal axis (x) thereof, so that its interior defines an inductive cylindrical measuring volume (Ev), wherein the half coils (Ela, E1b) together with a capacitance (E2) electrically connected parallel thereto form a parallel resonant circuit (E6) which is used as an inductive sensor of the inductive measuring system (E) which is connected to an electronic evaluation circuit (E5), and that the optical disc-shaped measuring volume (DCPv) is defined by the distance of the half-coils (Ela, E1b) and the inner wall of the housing (2) and disposed inside the inductive cylindrical measuring volume (Ev).

2. Arrangement according to Claim 1, **characterized in that** the length of a half-coil (Ela, E1b) is at least half as large as the diameter thereof and that the inductive cylindrical measuring volume (Ev) is preferably limited by cable bushes (4a, 4b) and preferably limited radially.

3. Arrangement according to one of Claims 1 or 2, **characterized in that** the parallel resonant circuit (E6) of the inductive measuring system (E) is connected to an excitation circuit (E3), preferably operated at its natural frequency, and is connected to an electronic circuit (E5) for voltage amplitude measurement, or that the parallel resonant circuit (E6) of the inductive measuring system (E) is connected to a frequency generator and to an electronic circuit (E5) for measuring the amplitude response and/or the phase response.

4. Arrangement according to any one of Claims 1 to 3, **characterized in that** the optical disc-shaped measuring volume (DCPv) of the first optical measuring system (D) is positioned preferably centrally in the longitudinal direction and preferably coaxially in the inductive cylindrical measuring volume (Ev) which surrounds the object (W).

5. Arrangement according to any one of Claims 1 to 4, **characterized in that** the first optical measuring system (D) includes at least a first illumination arrangement (DP1) having at least one first light source (DP2), and preferably a diaphragm (DP3) and a first sensor array (DP4) positioned on the main axis (y) on the opposite side of the half coils (E1a, E1b), and preferably at least one second optical measuring system (C) is designed for determining the colour of the object (W) .

6. Arrangement according to Claim 5, **characterized in that** the second optical measuring system (C) has a second illumination arrangement (C1) with a plurality, preferably three and preferably also closely placed light sources (C1a, C1b, C1c) with different wavelength spectra and at least a second sensor array (C4) for the light reflected from the object (W), wherein the second sensor array (C4) is located on the same side of the disc-shaped measuring volume (DCPv) as the second illumination arrangement (C1) relative to the x-z plane.

7. Arrangement according to Claim 6, **characterized in that** in the second optical measuring system (C) a sequence is implemented to actuate the light sources (C1a, C1b, C1c) of the second lighting arrangement (C1) in such manner as to illuminate the object (W) sequentially and thus sequentially project images in the wavelength spectra of the light sources of the second illumination arrangement onto the second sensor array (C4), wherein said second sensor array is connected to an evaluation unit for the intensities measured when illuminating with the light sources of different wavelength spectra and for the consequent determination of the colour of the object (W).

8. Arrangement according to either of Claims 6 or 7, **characterized in that** a reflective long-pass filter (C3) is arranged on the main axis (y) of the first and second optical measuring systems (D, C), which is reflective for the wavelength spectrums of the second illumination arrangement (C1) and passable for those of the first illumination arrangement (DP1), and which deflects the light reflected from the object (W) onto the second sensor array (C4), which is positioned outside the main optical axis (y) and aligned with the long-pass filter (C3).

9. Arrangement according to Claim 8, **characterized in that** a lens (DCP2) is arranged between the disc-shaped measuring volume (DCPv) and the long-pass filter (C3), which lens is passed through once as collimation lens (DCP2) by the light from the first illumination arrangement (DP1) and a second time as imaging lens (DCP2) by light from the second lighting arrangement (C1) reflected by the object (W)

10. Arrangement according to any one of Claims 5 to 9, **characterized in that** the optical measuring systems (D) for determining the diameter and (C) for determining the colour of the object (W) are combined to form a third, virtual optical measuring system (P) for determining the position of the object (W) within the disc-shaped measuring volume (DCPv), wherein particularly the main plane (y-z) of the first, second and third optical measuring systems (D, C, P) is arranged with the main optical axis (y) perpendicular to the longitudinal axis (x) of the inductive measuring volume (Ev).

11. Arrangement according to Claim 10, **characterized in that** the third measuring system (P) comprises a third illumination arrangement (P1) with preferably two light sources (P1a, P1b), the first sensor array (DP4) and optionally the first illumination arrangement (DP1) for determining the position of the object (W) within the measuring volume (DCPv) by triangulation, wherein in particular the third illumination arrangement (P1) has a plurality of light sources each having different wavelength spectra and are designed to illuminate the object (W) sequentially and thus project an image onto the second sensor array (C4) sequentially in the wavelength spectra of the light sources.

12. Arrangement according to any one of Claims 5 to 11, **characterized in that** the measuring plane of the first sensor array (DP4) extends perpendicularly to the x-y plane, but intersects it at only a small angle ($\alpha$) to the x-axis.

13. Arrangement according to any one of Claims 6 to 12, **characterized in that** the second sensor array (C4) is a multichromatic sensor, and the light sources (C1a, C1b and C1c) of the second illumination arrangement (C1) are operated simultaneously or are replaced by a broadband or multiband second light source, and/or at least one of the light sources of the third illumination arrangement (P1) is replaced with a wideband or multiband light source.

14. Arrangement according to any one of Claims 1 to 13, **characterized in that** the arrangement has a temperature sensor (T).

15. Arrangement according to any one of Claims 1 to 15, **characterized in that** a correction process is implemented in the inductive measuring system (E) to add to the measured value thereof a correction factor as a function of the temperature and a correction factor as a function of the position of the object (W).

16. System for processing elongated objects (W) such as cables, wires or profiles, **characterized in that** the system has an input-side feed for the elongated objects (W) arranged coaxially with the lengthwise extension thereof, wherein the system includes an arrangement according to any one of Claims 1 to 15.

## Revendications

1. Dispositif de détection automatique sans contact d'objets allongés (W) comme des câbles, des fils ou des profilés, comportant au moins un système de mesure inductif (E) pour la détermination d'une caractéristique électromagnétique de l'objet (W) et au moins un premier système de mesure optique (D) pour l'objet (W) dans un boîtier commun (2), dans lequel au moins un premier système de mesure optique (D) est conçu pour déterminer le diamètre extérieur (Wdo) de l'objet (W), un volume de mesure optique en forme de disque (DCPv) étant défini, **caractérisé en ce que** le système de mesure inductif (E) est conçu sous forme d'un capteur de courant de Foucault pour la détermination d'une caractéristique électromagnétique de l'objet (W) et présente deux demi-bobines (Ela, E1B) branchées en série, orientées dans le même sens coaxial et qui présentent dans le sens axial un intervalle entre elles et entourent l'objet (W) coaxialement par rapport à l'axe longitudinal (X), de sorte que leur intérieur définit un volume de mesure inductif de forme cylindrique (Ev), les demi-bobines (Ela, E1B) constituant avec une capacité (E2) connectée électriquement par rapport à celles-ci, un circuit résonant parallèle (E6) utilisé comme capteur inductif du système de mesure inductif (E) et qui est connecté à un circuit d'exploitation électronique (E5), et que le volume de mesure optique en forme de disque (DCPv) est défini par l'intervalle entre les demi-bobines (Ela, E1B) et la paroi intérieure du boîtier (2) et est disposé dans le volume de mesure inductif de forme cylindrique (Ev).

2. Dispositif selon la revendication 1, **caractérisé en ce que** la longueur d'une demi-bobine (Ela, E1B) représente au moins la moitié de son diamètre et que le volume de mesure inductif cylindrique (Ev) est de de préférence restreint par des connecteurs de câbles (4a, 4b) et de préférence radialement.

3. Dispositif selon la revendication 1 ou 2, **caractérisé en ce que** le circuit résonant parallèle (E6) du système de mesure inductif (E) est connecté à un circuit excitateur (E3), fonctionnant de préférence à sa fréquence propre, et à un circuit électronique (E5) pour mesurer l'amplitude de tension, ou que le circuit résonant parallèle (E6) du système de mesure inductif (E) est connecté à un générateur de fréquence et à un circuit électronique (E5) pour mesurer la courbe d'amplitude et/ou la courbe de phase.

4. Dispositif selon une des revendications 1 à 3, **caractérisé en ce que** le volume de mesure optique en forme de disque (DCPv) du premier système de mesure optique (D) est positionné de préférence dans le sens longitudinal au milieu et de préférence coaxialement dans le volume de mesure inductif de forme cylindrique (Ev) entourant l'objet (W).

5. Dispositif selon une des revendications 1 à 4, **caractérisé en ce que** le premier système de mesure optique (D) présente au moins un premier dispositif d'éclairage (DP1) comportant au moins une première source lumineuse (DP2) et de préférence un écran (DP3), de même qu'un premier réseau de capteurs (DP4) positionné au-delà des demi-bobines (Ela, E1B) sur l'axe principal (y) et qu'au moins un deuxième système de mesure optique (C) permettant de déterminer la couleur de l'objet (W) est de préférence réalisé.

6. Dispositif selon la revendication 5, **caractérisé en ce que** le deuxième système de mesure optique (C) présente un deuxième dispositif d'éclairage (C1) comportant plusieurs sources lumineuses (C1a, C1b, C1c), de préférence trois, également de préférence placées les unes près des autres et ayant des spectres de longueurs d'ondes différents, de même qu'au moins un second réseau de capteurs (C4) pour la lumière réfléchie par l'objet (W), le second réseau de capteurs (C4) se trouvant, par rapport au plan x-z, sur le même côté du volume de mesure optique en forme de disque (DCPv) que le deuxième dispositif d'éclairage (C1).

7. Dispositif selon la revendication 6, **caractérisé en ce que,** dans le second dispositif de mesure optique (C), est mis en œuvre un cycle consistant à commander les sources lumineuses (C1a, C1b, C1c) du deuxième dispositif d'éclairage (C1) de manière à éclairer l'objet (W) séquentiellement et ainsi à projeter séquentiellement des représentations dans les spectres de longueur d'onde des sources lumineuses du deuxième dispositif d'éclairage sur le second réseau de capteurs (C4), celui-ci étant connecté à une unité d'exploitation pour les intensités mesurées lors de l'éclairage avec les sources lumineuses à spectres de longueurs d'ondes différents et pour la détermination consécutive de la couleur de l'objet (W).

8. Dispositif selon la revendication 6 ou 7, **caractérisé en ce que,** sur l'axe principal (y) des premier et second systèmes de mesure optique (D, C), est disposé un filtre passe-long réfléchissant (C3) qui est réfléchissant pour les spectres de longueurs d'ondes du deuxième dispositif d'éclairage (C1) et est transparent pour ceux du premier dispositif d'éclairage (DP1) et qui dévie la lumière réfléchie par l'objet (W) sur le second réseau de capteurs (C4) positionné à l'extérieur de l'axe optique principal (y) et orienté en direction du filtre passe-long (C3).

9. Dispositif selon la revendication 8, **caractérisé en ce que,** entre le volume de mesure optique en forme de disque (DCPv) et le filtre passe-long (C3), est disposée une lentille (DCP2) qui est traversée aussi bien une fois comme lentille de collimation (DCP2) par la lumière du premier dispositif d'éclairage (DP1) que deux fois comme lentille éclairante (DCP2) par la lumière réfléchie par l'objet (W) du deuxième dispositif d'éclairage (C1).

10. Dispositif selon une des revendications 1 à 9, **caractérisé en ce que** les systèmes de mesure optiques (D) sont combinés pour déterminer le diamètre et (C) pour déterminer la couleur de l'objet (W) pour un troisième système de mesure optique virtuel (P) afin de déterminer la position de l'objet (W) dans le volume de mesure optique en forme de disque (DCPv), en particulier le plan principal (y-z) des premier, deuxième et troisième systèmes de mesure optiques (D, C, P) étant disposé avec l'axe optique principal (y) perpendiculairement à l'axe longitudinal (x) du volume de mesure inductif (Ev).

11. Dispositif selon la revendication 1, **caractérisé en ce que** le troisième système de mesure (P) comprend un troisième dispositif d'éclairage (P1) comportant de préférence deux sources lumineuses (P1a, P1b), le premier réseau de capteurs (DP4) et en option le premier dispositif d'éclairage (DP1) pour la détermination triangulaire de la position de l'objet (W) dans le volume de mesure (DCPv), le troisième dispositif d'éclairage (P1) présentant en particulier plusieurs sources lumineuses ayant respectivement des spectres de longueurs d'ondes différents et étant conçu pour éclairer l'objet (W) séquentiellement et projeter ainsi séquentiellement une représentation dans les spectres de longueurs d'ondes des sources lumineuses sur le second réseau de capteurs (C4).

12. Dispositif selon une des revendications 5 à 11, **caractérisé en ce que** le plan de mesure du premier réseau de capteurs (DP4) s'étend perpendiculairement au plan x-y mais coupe celui-ci suivant un angle aigu ($\alpha$) par rapport à l'axe x.

13. Dispositif selon une des revendications 6 à 1, **caractérisé en ce que** le second réseau de capteurs (C4) est un capteur multi-chromatique et que les sources lumineuses (C1a, C1b et C1c) du deuxième dispositif d'éclairage (C1) fonctionnent simultanément ou sont remplacées par une seconde source lumineuse à large bande ou à bandes multiples, et/ou qu'au moins une des sources lumineuses du troisième dispositif d'éclairage (P1) est remplacée par une seconde source lumineuse à large bande ou à bandes multiples.

14. Dispositif selon une des revendications 1 à 13, **caractérisé en ce que** le dispositif présente un capteur de température (T)

**15.** Dispositif selon une des revendications 1 à 14, **caractérisé en ce qu'**un cycle de correction est mis en œuvre dans le système de mesure inductif (E) afin de pourvoir sa valeur de mesure d'un facteur correctif en fonction de la température et d'un facteur correctif en fonction de la position de l'objet (W).

**16.** Installation d'usinage d'objets allongés (W) comme des câbles, des fils ou des profilés, **caractérisée en ce que** l'installation présente un système d'acheminement situé au niveau de l'entrée pour les objets allongés (W) coaxialement rapport à son extension longitudinale, l'installation présentant un dispositif selon une des revendications 1 à 15.

FIG 1

FIG 2

Draufsicht

EP 3 436 767 B1

**FIG 3**

**FIG 4**

EP 3 436 767 B1

# FIG 5

# FIG 6

## FIG 7

E1b

C1a
C1 { C1b
C1c

E1a

W

4b

X

4a

2

## FIG 8

Analoges
Ausgangssignal
des Pixelarray

Lichtstrahl

W

S1

DP4, Pixelarray
(Linearsensorarray)

FIG 9

## FIG 10

C1a, C1b, C1c

C1

C3

DCP2

3

hinten

oben

2

S1

S2

vorne

DP4

W

Y

P1b

P1a

4a

unten

## FIG 11

H' H

L₁        L₂

G

F₁   F₂        F_S              F'_S          F'₁   F'₂

B

FFL_S

f_S

f'_S

f₂

d        BFL_S

f₁

## FIG 12

## FIG 13

Objektabstand g, Bildabstand b und Vergrösserung M
gegen Objekt-Bild-Abstand c
g, b, M = f(c)

b, g (mm) und M (%)

Objekt-Bild-Abstand c (mm)

## FIG 14

RGB Werte, 2.65; 6.75; 4.75 ms Blitz, unterschiedliche Positionen

RGB Werte hinten/oben

RGB Werte mittig/oben

Blau Grün Rot

RGB Werte hinten/mittig

RGB Werte mittig/mittig

RGB Werte vorne/mittig

Hinten = Kalibrierstab nahe DP4

RGB Werte mittig/unten

## FIG 15

RGB Werte, 2.65; 6.75; 4.75 ms Blitz, oranges Kabel

RGB Werte bei Weissabgleich

Kabeldurchmesser: 2.1 mm
Linearsensorarray: 400 dpi

Rot-Werte eines orangen Kabels

Grün-Werte eines orangen Kabels

Blau-Werte eines orangen Kabels

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10219848 A1
- WO 2009150620 A1
- CN 1403821 A
- EP 0692697 A2
- GB 2160654 A
- JP 2000161985 A
- DE 102006010992 A1